Europäisches Patentamt

European Patent Office

Office européen des brevets

(11) Veröffentlichungsnummer: **0 191 935**
**B1**

(12) **EUROPÄISCHE PATENTSCHRIFT**

(45) Veröffentlichungstag der Patentschrift:
**05.10.88**

(21) Anmeldenummer: **85116054.9**

(22) Anmeldetag: **17.12.85**

(51) Int. Cl.⁴: **C 07 C 47/12,** C 07 C 45/50,
C 07 C 45/42

(54) **Verfahren zur Herstellung von 2-Alkyl-1,4-butandial.**

(30) Priorität: **19.02.85 DE 3505654**

(43) Veröffentlichungstag der Anmeldung:
**27.08.86 Patentblatt 86/35**

(45) Bekanntmachung des Hinweises auf die Patenterteilung:
**05.10.88 Patentblatt 88/40**

(84) Benannte Vertragsstaaten:
**AT BE CH DE FR GB IT LI NL SE**

(56) Entgegenhaltungen:
**DE - A - 3 403 427**

(73) Patentinhaber: **Degussa Aktiengesellschaft,
Weissfrauenstrasse 9, D-6000 Frankfurt am Main 1 (DE)**

(72) Erfinder: **Andrade, Juan, Dr. Dipl.-Chem., Hungerweg 5,
D-8752 Kleinostheim (DE)**
Erfinder: **Prescher, Günter, Dr. Dipl.-Chem.,
Liesingstrasse 2, D-6450 Hanau 9 (DE)**
Erfinder: **Samson, Marc, Dr. Dipl.-Chem., A.
Vermeylenstraat 2, B-9110 Lokeren (BE)**

## Beschreibung

Die Erfindung betrifft ein Verfahren zur Herstellung von 2-Alkyl-1,4-butandial.

Es ist bekannt, 3-Alkyl-4,4-dialkoxybutanole durch Hydroformylierung von α,β-ungesättigten Aldehydacetalen unter Verwendung eines Katalysators der Formel RhCl(Co)(PPh₃)₂ herzustellen.

Die Umsetzung wird in Benzol als Lösungsmittel und in Gegenwart von Triethylamin durchgeführt, das Nebenreaktionen vermeiden helfen soll (C. Botteghi et al., J. Org. Chem. 58 (1973) 2361–2365; Chim. Ind (Milan) 52, (1970) 265).

Andere Triarylphosphin/Rhodium/Carbonyl-Komplexkatalysatoren sind gemäss DE-AS-1 793 069 ebenfalls (=US-PS-3 527 809) geeignet, die Hydroformylierung von α-olefinischen Verbindungen zu Aldehyden zu katalysieren. Nach diesem Verfahren muss man jedoch die Bildung von Gemischen aus n- und iso-Aldehyden in Kauf nehmen. Die Verwendung eines Lösungsmittels führt unter ansonsten gleichen Bedingungen zu einer Verringerung des n-Aldehydanteils.

Die erfindungsgemäss hergestellten Dialdehyde sind als Härtungskomponente in Entwicklerlösungen geeignet (Research Disclosure, April 1981, 149).

Aufgabe der Erfindung ist ein Verfahren zur Herstellung von 2-Alkyl-1,4-butandial, das ohne Verwendung von Triethylamin und Lösungsmittel zu einheitlichen Produkten führt.

Gegenstand der Erfindung ist ein Verfahren zur Herstellung von 2-Alkyl-1,4-butandial, bei dem man das Acetal eines 2-Alkyl-substituierten 1-Propenals (2-Alkyl-3,3-dialkoxy-1-propen) in Gegenwart eines Komplexkatalysators, enthaltend Rhodium und dreiwertige organische Phosphorverbindungen als Liganden hydroformyliert, dadurch gekennzeichnet, dass man

(1) als Katalysator Hydridotris-triphenyl-phosphin-rhodiumcarbonyl gemeinsam mit Triphenylphosphin und/oder Triphenylphosphit einsetzt

(2) aus dem Hydroformylierungsprodukt das 3-Alkyl-4,4-dialkoxybutanal abtrennt und

(3) dieses zum 2-Alkyl-1,4-butandial hydrolysiert.

Dieses Verfahren führt ohne Verwendung von Lösungsmitteln und Triethylamin in hohen Ausbeuten zu dem gewünschten Produkt, ohne dass Ausbeuteverluste durch Isomerenbildung festgestellt werden.

Zur Durchführung des erfindungsgemässen Verfahrens wird zunächst das 2-Alkyl-1-propenal mit einem Alkanol zu einem 2-Alkyl-3,3-dialkoxy-1-propen umgesetzt. Vorzugweise wird ein 2-Alkyl-3,3-dialkoxy-1-propen gewählt, das in jeder Alkoxy-Gruppe 1 bis 6 Kohlenstoffatome aufweist. Besonders geeignet sind 2-Alkyl-3,3-dimethoxy-1-propen und 2-Alkyl-3,3-diäthoxy-1-propen. Die Umsetzung erfolgt in bekannter Weise, zum Beispiel nach dem in Org. Synth. Coll. Vol. 4 (1963), Seiten 21 bis 22, beschriebenen Verfahren durch Einwirkung der Orthoameisensäureester der entsprechenden Alkanole auf Acrolein oder nach dem in der DE-PS-930 752 oder der US-PS-

2 626 283 beschriebenen Verfahren durch Einwirkung der betreffenden Alkanole auf den Aldehyd in Gegenwart von starken Säuren als Katalysatoren.

Mit Vorteil wird für die Herstellung von 2-Alkyl-3,3-dimethoxy-1-propens und 2-Alkyl-3,3-diäthoxy-1-propens das Verfahren gemäss der DE-OS-3 403 426 angewendet. Die in 2-Stellung substituierte Alkylgruppe besitzt 1 bis 4 Kohlenstoffatome und ist verzweigt oder geradkettig.

Bevorzugt sind die Methyl-, Ethyl-, n-Propyl-, iso-Propyl, n-Butyl und iso-Butyl-Gruppe.

Zur weiteren Durchführung des erfindungsgemässen Verfahrens wird das 2-Alkyl-3,3-dialkoxy-1-propen durch Umsetzung mit einem Gasgemisch aus Wasserstoff und Kohlenmonoxid in Gegenwart des Katalysators hydroformyliert. Die Umsetzung erfolgt zweckmässigerweise bei erhöhter Temperatur, vorzugsweise bei Temperaturen von 100 bis 140 °C. Der Druck kann weitgehend beliebig gewählt werden, jedoch ist es im allgemeinen zweckmässig, wenigstens bei Normaldruck zu arbeiten. Bevorzugt werden Drücke von 1 bis 60 bar. Zweckmässig ist es, mindestens stöchiometrische Mengen, vorzugsweise überschüssige Mengen, Wasserstoff und Kohlenmonoxid anzuwenden, wobei das Molverhältnis Wasserstoff zu Kohlenmonoxid weitgehend beliebig gewählt werden kann, vorzugsweise jedoch von 0,5 zu 1,0 bis 1,0 zu 0,5 reicht.

Bei der Hydroformylierung dient als Katalysator Hydridotris-triphenylphosphin-rhodiumcarbonyl im Gemisch mit Triphenylphosphin und beziehungsweise oder Triphenylphosphit. Derartige Katalysatoren sind in der DE-AS-1 793 069 beschrieben. Vorzugsweise werden bei der Durchführung des erfindungsgemässen Verfahrens je Gewichtsteil des 2-Alkyl-3,3-Dialkoxy-1-propens etwa 0,0001 bis 0,0070 Gewichtsteile des Hydridotris-triphenylphosphin-rhodiumcarbonyls und 0,001–0,06 Gewichtsteile des Triphenylphosphins oder Triphenylphosphits angewendet.

Das Hydroformulierungsprodukt wird zur Gewinnung des 3-Alkyl-4,4-Dialkoxybutanals fraktionierend destilliert. Hierbei wird zweckmässigerweise unter vermindertem Druck gearbeitet, vorzugsweise bei Drücken unter 40 mbar.

Das so gewonnene 3-Alkyl-4,4-dialkoxybutanal wird schliesslich durch Hydrolyse in das 2-Alkyl-1,4-butandial übergeführt. Die Hydrolyse erfolgt in saurem Medium, vorzugsweise in stark saurem Medium, mit besonderem Vorteil in Gegenwart eines sauren Ionenaustauschers, zweckmässigerweise bei Temperaturen unter 30 °C, vorzugsweise bei Temperaturen zwischen 5 und 15 °C.

Beispiel 1

2-Methyl-3,3-dimethoxy-1-propen (263 g) wurde in einem Rührautoklaven mit 2,6 g Triphenylphosphit und 0,4 g Hydrodotris-triphenylphosphin-rhodiumcarbonyl vorgelegt. Dann wurde unter 6 bar Druck eine Mischung aus gleichen Volumenteilen Wasserstoff und Kohlenmonoxid eingespeist. Die Temperatur im Autoklaven wurde hierbei auf 115 °C gehalten. Nach 150 min wurde kein Gas mehr aufgenommen und die Einspeisung beendet. Die gaschromatographische Untersuchung erbrachte, dass 99% des 2-Methyl-3,3-dimethoxy-1-propens umgesetzt waren. Das Umsetzungsgemisch, das 94% 3-Methyl-4,4-dimethoxy-butanal enthielt, wurde bei 20 mbar destilliert. Das gewünschte 3-Methyl-4,4-dimethoxybutanal ging bei 75 °C über. Es wurde in gleichen Volumenteilen Wasser gelöst, die Lösung mit 60 g des Kationenaustauschers Dowex® MSC-1 Z (Polystyrolbasis, aktive Gruppe: Sulfonsäure) versetzt, diese Mischung 2 h lang bei 20 °C gerührt und dann filtriert. Als Filtrat erhielt man 32%ige wässrige Lösung des 2-Methyl-1,4-Butandials. Die Ausbeute an 1,4-Butandial, bezogen auf das eingesetzte 2-Methyl-3,3-dimethoxy-1-propen, betrug 94%.

Beispiel 2

Es wurde wie nach Beispiel 1 verfahren, jedoch wurden 232 g (2,0 mol) des 2-Methyl-3,3-dimethoxy-1-propens mit 0,71 g Triphenylphosphit und 0,3 g Hydridotris-triphenylphosphin-rhodiumcarbonyl vorgelegt, und die Hydroformylierung erfolgte bei 30 bar und 130 °C. 99,8% des 2-Methyl-3,3-dimethoxy-1-propens wurden umgesetzt. Das Umsetzungsgemisch enthielt 97,4% 3-Methyl-4,4-dimethoxybutanal. Die Ausbeute an 2-Methyl-1,4-butandial, bezogen auf das eingesetzte 2-Methyl-3,3-dimethoxy-1-propen, betrug 97%.

Beispiel 3

Es wurde wie nach Beispiel 1 verfahren, jedoch wurden 350 g (ca. 3,0 mol) des 2-Methyl-3,3-dimethoxy-1-propens mit 6,0 g Triphenylphosphin und 0,7 g Hydridotris-triphenylphosphin-rhodiumcarbonyl vorgelegt, und die Hydroformylierung erfolgte bei 50 bar und 105 °C. 99,9% des 2-Methyl-3,3-dimethoxy-1-propens wurden umgesetzt. Das Umsetzungsgemisch enthielt 98% 3-Methyl-4,4-dimethoxybutanal. Die Ausbeute an 2-Methyl-1,4-butandial bezogen auf das eingesetzte 2-Methyl-3,3-dimethoxy-1-propen betrug 97%.

Beispiel 4

Es wurde wie nach Beispiel 1 verfahren, jedoch wurden 200,0 g (1,41 mol) des 2-Ethyl-3,3-dimethoxy-1-propens mit 1,0 g Triphenylphosphit und 0,6 g Hydridotris-triphenylphosphin-rhodiumcarbonyl vorgelegt, und die Hydroformylierung erfolgte bei 8 bar und 119 °C; die Gasaufnahme war nach 160 min beendet. 81% des 2-Ethyl-3,3-dimethoxy-1-propens wurden umgesetzt. Das Umsetzungsgemisch wurde im Vakuum destilliert. Das gewünschte 3-Ethyl-4,4-dimethoxybutanal ging bei 85 °C und 20 mbar über. Das 2-Ethyl-1,4-Butandial wurde dann wie im Beispiel 1 gewonnen.

Beispiel 5

Es wurde wie nach Beispiel 1 verfahren, jedoch wurden 200 g (1,39 mol) des 2-n-Propyl-3,3-dimethoxy-1-propens mit 1,3 g Triphenylphosphit und 0,5 g Hydridotris-triphosphin-rhodiumcarbonyl vorgelegt, und die Hydroformylierung erfolgte bei 6 bar und 127 °C; die Gasaufnahme war nach 180 min beendet. 85% des 2-n-Propyl-3,3-dimethoxy-1-propens wurden umgesetzt. Das Umsetzungsgemisch wurde im Vakuum destilliert. Das gewünschte 3-n-Propyl-4,4-dimethoxybutanal ging bei 100 bis 102 °C und 20 mbar über. Das 2-n-Propyl-1,4-butandial wurde dann wie in Beispiel 1 gewonnen.

Beispiel 6

Es wurde wie nach Beispiel 1 verfahren, jedoch mit 200 g (1,39 mol) des 2-iso-Propyl-3,3-dimethoxy-1-propens. Nach 300 min waren 50% des 2-iso-Propyl-3,3-dimethoxy-1-propens umgesetzt. Das gewünschte 3-iso-propyl-4,4-Dimethoxybutanal ging bei 94 bis 96 °C und 20 mbar über. Das 2-iso-Propyl-1,4-butandial wurde dann wie in Beispiel 1 gewonnen.

**Patentansprüche**

1. Verfahren zur Herstellung von 2-Alkyl-1,4-butandial, bei dem man 2-Alkyl-3,3-dialkoxy-1-propen in Gegenwart eines Komplexkatalysators, enthaltend Rhodium und dreiwertige organische Phosphorverbindungen als Liganden hydroformyliert, dadurch gekennzeichnet, dass man
(1) als Katalysator Hydridotris-triphenylphosphin-rhodiumcarbonyl gemeinsam mit Triphenylphosphin und/oder Triphenylphosphit einsetzt
(2) aus dem Hydroformylierungsprodukt das 3-Alkyl-4,4-dialkoxybutanal abtrennt und
(3) dieses zum 2-Alkyl-1,4-butandial hydrolysiert.

2. Verfahren nach Anspruch 1, dadurch gekennzeichnet, dass man ein 3,3-Dialkoxy-1-propen einsetzt, das in 2-Stellung mit einer geradkettigen oder verzweigten $C_1$–$C_4$-Alkylgruppe substituiert ist.

3. Verfahren nach den Ansprüchen 1 und 2, dadurch gekennzeichnet, dass man je Gewichtsteil des 3,3-Dialkoxy-1-propens von 0,0001–0,0070 Gewichtsteile des Hydridotris-triphenylphosphin-rhodiumcarbonyls und 0,001–0,06 Gewichtsteile des Triphenylphosphins oder Triphenylphosphits anwendet.

4. Verfahren nach den Ansprüchen 1 bis 3, dadurch gekennzeichnet, dass man die Hydroformylierung bei Temperaturen von 100 bis 140 °C und Drücken von 1 bis 60 bar durchführt.

**Claims**

1. Process for the preparation of 2-alkyl-butane-1,4-dial wherein 2-alkyl-3,3-dialkoxy-1-propene is hydroformylated in the presence of a complex catalyst containing rhodium and trivalent organic phosphorus compounds as ligands, characterized in that
(1) the catalyst used is hydridotris-triphenylphosphine-rhodium-carbonyl conjointly with triphenylphosphine and/or triphenylphosphite,
(2) the 3-alkyl-4,4-dialkoxybutanal is isolated from the hydroformylation product and
(3) is hydrolysed to give the 2-alkyl-butane-1,4-dial.

2. Process according to claim 1, characterized in that a 3,3-dialkoxy-1-propene which is substituted in the 2-position by a straight-chain or branched $C_1$–$C_4$-alkyl group is employed.

3. Process according to claims 1 and 2, characterized in that per part by weight of the 3,3-dialkoxy-1-propene, 0,0001–0,0070 part by weight of the hydridotris-triphenylphosphine-rhodium-carbonyl and 0,001–0,06 part by weight of the triphenylphosphine or triphenylphosphite are employed.

4. Process according to claims 1 to 3, characterized in that the hydroformylation is carried out at temperatures of 100 to 140 °C and pressures of 1 to 60 bar.


**Revendications**

1. Procédé d'obtention de 2-alcoyl-butane-1,4-dial dans lequel on hydroformyle un 2-alcoyl-3,3-dialcoxy-prop-1-ène en présence d'un catalyseur complexe contenant du Rhodium et des composés phosphorés organiques trivalents comme ligands, caractérisé en ce que:

(1) comme catalyseur on met en œuvre de l'hydridotris-triphénylphosphine-rhodiumcarbonyl ensemble avec de la triphénylphosphine et/ou du phosphite de Triphényle

(2) on sépare du produit d'hydroformylation le 3-alcoyl-4,4-dialcoxybutanal et

(3) hydrolyse celui-ci en 2-alcoyl-butane-1,4-dial.

2. Procédé selon la revendication 1, caractérisé en ce que l'on met en œuvre un 3,3-dialcoxy-prop-1-ène qui est substitué en position 2 par un groupe alcoyle en $C_1$–$C_4$ en chaîne droite ou ramifiée.

3. Procédé selon les revendications 1 et 2, caractérisé en ce qu'on utilise chaque fois une partie pondérale de 3,3-dialcoxy-prop-1-ène à partir de 0,0001–0,0070 de partie pondérale d'Hydridotris-triphénylphosphine-rhodiumcarbonyl et 0,001–0,06 de partie pondérale de Triphénylphosphine ou de Triphénylphosphite.

4. Procédé selon les revendications 1 à 3, caractérisé en ce qu'on conduit l'hydroformylation à une température comprise entre 100 et 140 °C et une pression comprise entre 1 à 60 bars.